# DEMANDE DE BREVET EUROPEEN

(11) **EP 1 219 254 A1**
(43) Date de publication de la demande: **03.07.2002**
(21) Numéro de dépôt: 00811254.2
(22) Date de dépôt: 28.12.2000
(51) Int. Cl.: A61B 17/54

(54) **Dispositif de dermabrasion**

(71) Demandeur: Bernaz, Gabriel, CH-1227 Carouge (CH)
(72) Inventeur: Bernaz, Gabriel, CH-1227 Carouge (CH)
(74) Mandataire: Cronin, Brian Harold John

(57) **Abrégé**

Dispositif de dermabrasion de la peau par effleurage de la peau avec un abrasif comportant un boîtier (10) maniable et des moyens d'entraînement de l'abrasif. Ce dispositif est destiné en particulier aux traitements cosmétiques de l'épiderme appelés microépidermabrasion. Le dispositif comporte une surface abrasive arquée (30) tenue par un support (32) monté dans ou sur le boîtier (10, 18) pour un mouvement oscillant permettant l'oscillation de la surface abrasive arquée (30) autour de son axe. Une surface d'appui (40) entoure au moins partiellement la surface abrasive oscillante de manière à permettre, par l'application de la surface d'appui (44) contre la peau (50), l'effleurage de la peau par la surface abrasive oscillante. On obtient ainsi une microabrasion de l'épiderme par l'oscillation de la surface abrasive arquée (30) appliquée légèrement contre la peau grâce à la surface d'appui (40) qui empêche toute blessure ou pénétration au-delà de l'épiderme.

## Description

### DOMAINE TECHNIQUE

L'invention se rapporte à la dermabrasion de la peau par effleurage de la peau avec un abrasif, en particulier pour la microépidermabrasion de la peau, c'est-à-dire la microabrasion de la couche superficielle de la peau, ou épiderme, qui protège la partie profonde de la peau, ou derme, formée de tissu conjonctif contenant des vaisseaux, des nerfs et des follicules.

### ETAT DE LA TECHNIQUE

La microdermabrasion par la projection des microcristaux d'hydroxyde d'aluminium est connue par exemple par "La microdermabrasion en pratique" par François Mahuzier (http://www.mahuzier.com) ou encore par "La Dermabrasion" par Dr. André Camirand (http://www.drcamirand.com), ou encore par "Dermabrasion & Dermaplaning" par la Société canadienne de chirurgie plastique et esthétique (http://www.smartnet.ca). Ces articles mentionnent aussi la dermabrasion par des grattements avec une brosse métallique, ou par un burin contenant des particules de diamant, alors que le dermaplaning emploie un instrument appelé dermatome qui comporte une lame oscillante pour déloger des couches de surface de la peau.

La microdermabrasion par la projection des microcristaux de corundum est également décrite dans la publication EP-A-0 806 184.

La microdermabrasion par la projection d'un liquide est connue du brevet US 5,562,643, alors que le brevet US 5,562,643 décrit la microdermabrasion par l'emploi d'un outil à ultrasons, après l'application d'un anesthésique.

Le brevet US 5,562,643 décrit un appareil pour la microdermabrasion chirurgicale comportant une pluralité de feuilles flexibles à bord abrasif tournant autour d'un moyeu pour venir frapper la peau par la force centrifuge.

Ces appareils et procédés de dermabrasion sont destinés essentiellement à l'abrasion de la peau jusqu'au derme, et nécessitent en général une intervention chirurgicale.

Le traitement cosmétique de l'épiderme utilisant un tampon abrasif maniable du type Scotch-Brite™ est connu du brevet US 6,017,351, principalement dans le but d'enlever un excédant de peau morte.

Le brevet US 4,438,767 décrit une lame maniable d'exfoliation, également pour enlever un excédant de peau morte.

### EXPOSE DE L'INVENTION

La présente invention a pour objet un dispositif de dermabrasion de la peau par effleurage de la peau avec un abrasif, le dispositif comportant un boîtier maniable et des moyens d'entraînement de l'abrasif. Ce dispositif est destiné en particulier aux traitements cosmétiques de l'épiderme appelés microépidermabrasion.

Le dispositif selon l'invention est caractérisé par le fait qu'il comporte, en combinaison, une surface abrasive arquée tenue par un support monté dans ou sur le boîtier pour un mouvement oscillant permettant l'oscillation de la surface abrasive arquée autour de son axe, et une surface d'appui entourant au moins partiellement la surface abrasive oscillante de manière à permettre, par l'application de la surface d'appui contre la peau, l'effleurage de la peau par la surface abrasive oscillante.

On obtient ainsi une microabrasion de l'épiderme par l'oscillation de la surface abrasive arquée appliquée légèrement contre la peau grâce à la surface d'appui qui empêche toute blessure ou pénétration au-delà de l'épiderme. Le dispositif est aussi convivial à manier qu'un rasoir électrique, et permet la microépidermabrasion cosmétique de la peau pour différents traitements. Dès lors, il présente des avantages substantiels par rapport aux appareils connus de dermabrasion dont l'utilisation est limitée aux interventions chirurgicales ou analogues, et qui agissent jusque dans le derme. Par rapport aux appareils manuels pour traiter l'épiderme, le nouvel appareil permet un traitement automatisé et facile sur de grandes surfaces comme sur de petites surfaces, et permet de réaliser des traitements antirides et autres qui n'étaient pas faisables avec les appareils connus.

Pour promouvoir l'effet de microépidermabrasion, la surface abrasive arquée ne fait pas saillie par rapport à la surface d'appui, donc elle est soit au niveau de cette surface d'appui, soit avantageusement disposée en retrait par rapport à la surface d'appui, par exemple de 0 à 2 mm en retrait, usuellement au moins environ 1 à 1.5 mm, alors que le jeu latéral de la surface arquée par rapport aux bords de la surface d'appui est usuellement environ 1-4 mm de chaque côté.

Avantageusement, la surface abrasive est portée sur une pièce amovible en matière rigide ou souple sur le support oscillant. Ainsi, le dispositif peut comporter plusieurs pièces interchangeables chacune portant une surface abrasive différente et/ou ayant des dimensions différentes. Par exemple, on peut disposer de plusieurs pièces interchangeables portant une surface abrasive identique, mais de différentes longueurs adaptées au traitement de la peau de différentes parties du corps, et/ou plusieurs pièces interchangeables portant des surfaces abrasives différentes destinées à produire une microabrasion plus ou moins prononcée.

La pièce porte-abrasif est soit en matière rigide, par exemple en matière plastique moulée, soit en matière souple par exemple une pièce en silicone souple permettant d'adapter la force d'application de l'abrasif sur la peau.

Dans une forme d'exécution, la surface d'appui est constituée par les bords d'un élément en U qui entourent la pièce portant la surface abrasive, cette pièce étant amovible par l'extrémité ouverte de l'élément en U de la surface d'appui, facilitant ainsi l'échange des pièces interchangeables. L'extrémité ouverte permet aussi l'inclinaison de l'appareil pour le traitement d'endroits enfoncés, comme des sillons de rides.

La surface d'appui du dispositif est normalement constituée soit par les bords d'un élément amovible sur le boîtier, soit par les bords du boîtier. Dans ce dernier cas, les bords de la surface d'appui constituant une partie intégrante du boîtier lequel est par exemple en matière thermoplastique moulée.

Un dispositif dont la surface d'appui est disposée sur un élément amovible présente la possibilité d'utiliser accessoirement le dispositif avec cet élément démonté pour le traitement des parties du corps ne nécessitant pas l'utilisation d'une surface d'appui. On peut citer notamment le manucure et le pédicure.

Avantageusement, les moyens d'entraînement de l'abrasif permettent la variation de la vitesse d'oscillation de la surface abrasive dont la vitesse d'oscillation peut être comprise entre 0.5 à 200 oscillations par seconde, usuellement entre 5-100 oscillations par seconde.

Dans une forme d'exécution, ces moyens d'entraînement comportent un étrier solidaire d'un levier monté pivotant dans le boîtier, l'étrier entourant un excentrique entraîné par l'arbre d'un moteur électrique, le support abrasif étant monté à l'extrémité pivotante du levier. Dans ce cas, le support abrasif oscille en balancier autour de son axe de pivotement, cet axe de pivotement étant de préférence disposé entre l'excentrique et l'extrémité pivotante du levier, de manière à ce que le rayon de pivotement soit réduit.

De manière générale, ce rayon de pivotement serait au maximum environ 20 mm, usuellement entre 1-1.5 mm. Pour assurer un bon effet de microépidermabrasion l'amplitude d'oscillation est usuellement au maximum environ 4 mm (2 mm de chaque côté).

Dans une autre forme d'exécution, l'abrasif est disposé sur un support cylindrique oscillé par des moyens adéquats. Une telle configuration cylindrique permet de développer des surfaces oscillantes de grande taille, augmentant les possibilités d'application du dispositif. Avec un support abrasif cylindrique il est en outre possible de disposer de plusieurs zones abrasives écartées les unes par rapport aux autres autour de la périphérie du cylindre, chaque zone correspondant à un angle d'oscillation du cylindre. Le cylindre est alors indexable pour permettre la sélection de la zone abrasive accessible par la surface d'appui.

L'invention concerne également un procédé de traitement cosmétique de la peau par microépidermabrasion, utilisant le dispositif selon l'invention, de préférence après avoir appliqué auparavant à la surface de la peau à traiter un produit de nettoyage. Cette microépidermabrasion en effet expose le tissu de la peau et rend ce tissu de la peau plus apte à suivre divers traitements.

Un procédé de traitement cosmétique de la peau selon l'invention comporte avantageusement au préalable une microépidermabrasion au moyen de l'appareil inventif, suivi de l'application sur l'épiderme ainsi traité d'un produit traitant qu'on fait pénétrer dans le tissu de la peau par l'application d'un flux d'énergie électromagnétique haute fréquence et/ou par l'application de rayonnement électromagnétique laser, par exemple selon les brevets EP 0573618 et 0773744 et la demande de brevet WO/99/49800, ou par une source de lumière "normale".

Ce traitement est avantageusement un traitement antirides, autour des yeux, sur le front, autour de la bouche, etc. mais d'autres traitements sont aussi possible par exemple un traitement d'épilation au-dessus des lèvres ou autour des sourcils ou sur des surfaces plus larges. D'autres traitements sont le traitement des taches, des vergetures, de l'acné et des cicatrices. Il convient aussi pour des traitements sur le cuir-chevelu, notamment pour y faire pénétrer des produits de repousse de cheveux.

Enfin, l'invention concerne aussi l'utilisation du dispositif pour la microépidermabrasion de la peau.

### BREVE DESCRIPTION DES DESSINS

D'autres caractéristiques de l'invention ressortiront à la lecture de la description qui suit, donnée à titre d'exemple, en se référant aux dessins dans lesquels :
la Figure 1 est une vue latérale en coupe axiale d'une forme d'exécution du dispositif selon l'invention;
la Figure 2 est une coupe selon la ligne II-II de la Figure 1; et
la Figure 3 est une coupe selon la ligne III-III de la Figure 2, montrant l'appareil en contact avec la peau.

### MODES DE REALISATION PREFERES

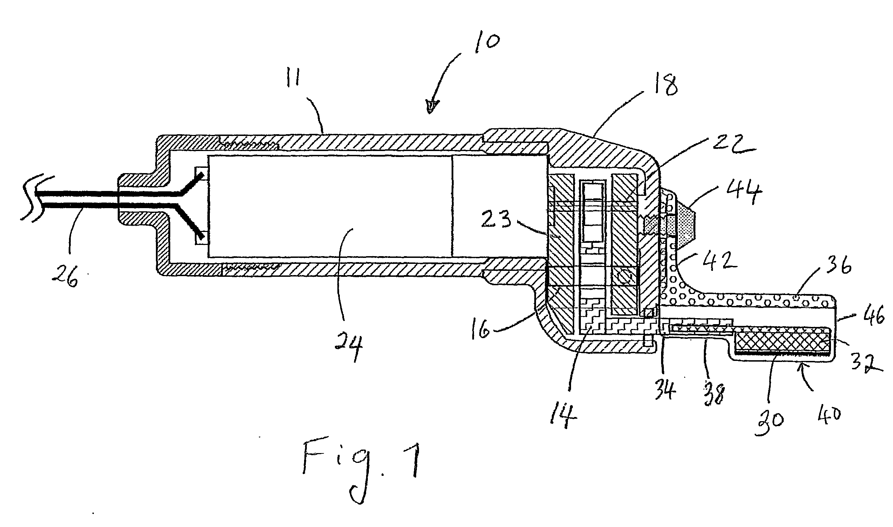

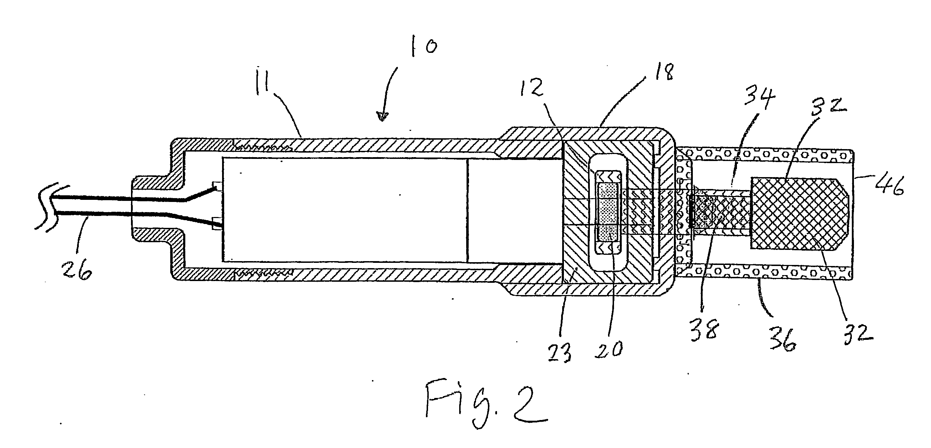

Les Figures 1 à 3 représentent un dispositif de dermabrasion de la peau comportant un boîtier maniable 10 et des moyens d'entraînement d'un abrasif 30. Dans cette forme d'exécution, les moyens d'entraînement comportent un étrier 12 solidaire d'un levier 14 monté pivotant autour d'un axe 16 dans un carter 18 monté à une extrémité du corps allongé du boîtier 10. Les deux bras de l'étrier 12 entourent un excentrique 20 entraîné par l'arbre 22 d'un moteur électrique 24 logé dans le corps allongé 11 du boîtier 10.

L'ensemble arbre 22, excentrique 20, levier 14, axe 16 est monté dans un corps monobloc 23 logé dans le carter 18.

La surface abrasive 30 est constituée d'une surface arquée d'une pièce allongée 32 retenue amoviblement sur un support 34 monté sur l'extrémité inférieure du levier 14, cette pièce 32 faisant saillie par l'extrémité avant du boîtier 10. L'axe de cette surface abrasive arquée 30 coïncide avec l'axe 16 de pivotement du levier 14. Ainsi, l'oscillation du levier 14 produit une oscillation en balancier de la surface abrasive arquée 30 autour de son axe dont le rayon de courbure se situe entre environ 1-3cm par exemple.

La pièce porte-abrasif 32 est par exemple en matière plastique moulée rigide, ou en silicone souple permettant d'adapter la force d'application de l'abrasif 30 sur la peau.

Le moteur 24 est alimenté par le secteur au moyen des fils 26 d'amenée de courant (et/ou par un accumulateur) et comporte un variateur de vitesse permettant de varier la vitesse de rotation de l'arbre 22 et excentrique 20, donc de la vitesse d'oscillation du levier 14 et de la surface abrasive 30. Cette surface abrasive 30 oscille de préférence à une vitesse entre 0.5 à 200 oscillations par seconde, variable progressivement ou par paliers, éventuellement à une seule vitesse d'oscillation dans cette gamme, usuellement entre 5 à 100 oscillations par seconde.

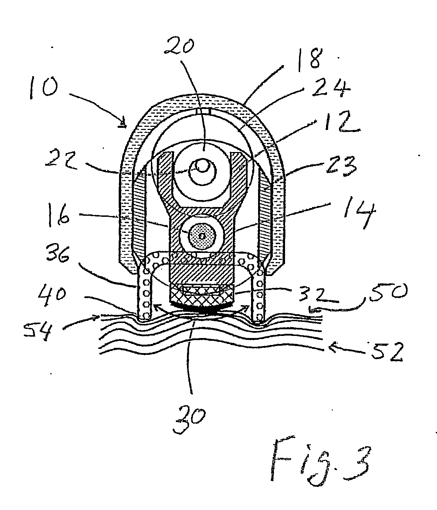

Le dispositif comporte en outre une surface d'appui 40 constituée par les bords d'un élément en U 36 qui entoure la pièce porte-abrasif 32. Cet élément 36 entoure trois côtés sur quatre de la surface abrasive oscillante 30 (voir la Figure 3). Cette pièce 32 comporte une tige 38 montée amoviblement par friction dans un logement correspondant du support oscillant 34.

L'élément en U 36 comporte une partie frontale 42 servant à solidariser cet élément 36 sur le carter 18 au moyen d'une vis 44, permettant aussi d'enlever cet élément 36 si désiré. Normalement cet élément 36 reste en place, car il est nécessaire pour la sécurité d'utilisation et pour assurer l'effleurage de l'abrasif oscillant sur la peau 50 suffisant pour effectuer une microépidermabrasion, sans risque de pénétration dans le derme 52.

Lorsque l'élément 36 est enlevé, il est encore possible d'utiliser le dispositif non plus pour la microépidermabrasion mais pour le traitement abrasif des parties du corps ne nécessitant pas la protection conférée par la surface d'appui 40, par exemple pour le manucure et le pédicure.

La pièce porte-abrasif 32 est montable et démontable par l'extrémité ouverte 46 de l'élément en U 36, facilitant ainsi l'échange des pièces 32 interchangeables. D'autres moyens de fixation amovible sont possible.

Le dispositif comporte avantageusement plusieurs pièces 32 interchangeables chacune portant une surface abrasive 30 différente, par exemple en poudre de saphir ou alumine, ou un autre abrasif ayant de manière générale une grosseur de grain usuellement dans la gamme 50-200, convenablement d'environ 80-120, et une dureté adaptées pour des traitements de microabrasion plus ou moins prononcée. De manière générale un abrasif d'un grain plus grossier ou plus fin sera utilisé avec une vitesse d'oscillation relativement basse ou haute, respectivement.

Les pièces porte-abrasifs 32 interchangeables peuvent aussi avoir des dimensions différentes, par exemple des longueurs différentes adaptées au traitement de la peau de différentes parties du corps.

Dans cet exemple, la surface abrasive arquée 30 est disposée en retrait par rapport à la surface d'appui 40, usuellement d'au moins environ 1 à 1.5 mm, alors que le jeu latéral de la surface arquée par rapport aux bords l'élément en U 36 est environ 1-2 mm de chaque côté.

L'utilisation de l'appareil est très commode car le boîtier 10 est maniable, comme un rasoir électrique. L'application de la surface d'appui 40 contre la peau 50 à traiter, permet l'effleurage de la peau 50 par la surface abrasive oscillante 30 entraîné par le moteur 24.

On obtient ainsi une microabrasion de l'épiderme 54 par l'oscillation de la surface abrasive arquée 30 appliquée légèrement contre la peau 50 grâce à la surface d'appui 40 qui empêche toute blessure ou pénétration au-delà de l'épiderme 54. De manière générale le dispositif est balayé sur la surface de la peau à traiter pendant un temps convenable, usuellement durant plusieurs minutes.

Normalement la surface arquée 30 sera appliquée à plat sur la peau 50, mais pour le traitement de sillons des rides et autres endroits enfoncés, il est possible d'incliner l'appareil (vers la droite de la Fig. 1) et travailler sur la pointe de l'abrasif, c'est-à-dire, l'extrémité à droite de la Fig. 1. L'angle de travail peut donc varier de 0° jusqu'à environ 45°.

Le dispositif est de préférence utilisé après avoir appliqué auparavant à la surface de la peau 50 à traiter une lotion détergent ou autre produit de nettoyage.

La microépidermabrasion au moyen de l'appareil peut être suivi de l'application sur l'épiderme 54 ainsi traité d'un gel ou autre produit traitant, par exemple un gel antirides qu'on fait pénétrer dans le tissu de la peau 50 par l'application d'un flux d'énergie électromagnétique haute fréquence et/ou par l'application de rayonnement électromagnétique laser cohérent, par exemple selon les brevets EP 0573618 et 0773744, ou de la lumière continue dans les couleurs bleu-vert-jaune-rouge de l'arc-en-ciel du spectre visible. Un traitement utilisant une source laser, par exemple selon la demande de brevet WO/9949800, et/ou un LED ou autre source de lumière colorée, est particulièrement commode, notamment un traitement avec l'application successive de deux ou plusieurs sources laser ou lumière à énergies différentes pendant plusieurs minutes. Un tel traitement antirides a donné des résultats spectaculaires après un temps de traitement total de 10 à 20 minutes.

D'autres applications sont le traitement des taches, des vergetures, de l'acné, des cicatrices, d'épilation ou du cuir-chevelu.

De manière générale, le traitement de microépidermabrasion améliore de manière significative des traitements ultérieurs. Pour des traitements sur de larges surfaces de la peau, il serait avantageux d'utiliser une surface abrasive disposée sur un cylindre oscillant.

Au lieu d'un moteur rotatif, le moyen d'entraînement pourrait comporter un moteur va-et-vient, comme un vibreur.

## Revendications

1. Dispositif de dermabrasion de la peau par effleurage de la peau avec un abrasif, le dispositif comportant un boîtier maniable (10) et des moyens d'entraînement de l'abrasif, **caractérisé par le fait qu'**il comporte, en combinaison, une surface abrasive arquée (30) tenue par un support (34) monté dans ou sur le boîtier (10, 18) pour un mouvement oscillant permettant l'oscillation de la surface abrasive arquée (30) autour de son axe (16), et une surface d'appui (40) entourant au moins partiellement la surface abrasive oscillante (30) de manière à permettre, par l'application de la surface d'appui (40) contre la peau (50), l'effleurage de la peau par la surface abrasive oscillante (30).

2. Dispositif de dermabrasion selon la revendication 1, **caractérisé en ce que** la surface abrasive arquée (30) est au niveau de la surface d'appui (40) ou en retrait par rapport à cette surface.

3. Dispositif de dermabrasion selon la revendication 1 ou 2, **caractérisé en ce que** la surface abrasive (30) est portée sur une pièce (32) en matière rigide ou souple montée amoviblement sur le support oscillant (34).

4. Dispositif de dermabrasion selon la revendication 3, **caractérisé en ce qu'**il comporte plusieurs pièces (32) interchangeables chacune portant une surface abrasive (30) différente et/ou ayant des dimensions différentes.

5. Dispositif de dermabrasion selon la revendication 3 ou 4, **caractérisé en ce que** la surface d'appui (40) est constituée par les bords d'un élément en U (36) qui entourent la pièce (32) portant la surface abrasive (30), cette pièce (32) étant amovible par l'extrémité ouverte de l'élément en U (40) de la surface d'appui.

6. Dispositif de dermabrasion selon l'une quelconque des revendications précédentes, **caractérisé en ce que** ladite surface d'appui (40) est constituée par les bords d'un élément (36) monté amoviblement sur le boîtier.

7. Dispositif de dermabrasion selon l'une quelconque des revendications 1 à 5, **caractérisé en ce que** ladite surface d'appui (40) est constituée par les bords du boîtier.

8. Dispositif de dermabrasion selon l'une quelconque des revendications précédentes, **caractérisé en ce que** les moyens d'entraînement (24) permettent la variation de la vitesse d'oscillation de la surface abrasive oscillante (30).

9. Dispositif de dermabrasion selon l'une quelconque des revendications précédentes, **caractérisé en ce que** la surface abrasive oscillante (30) a une vitesse d'oscillation entre 0.5 à 200 oscillations par seconde.

10. Dispositif de dermabrasion selon l'une quelconque des revendications précédentes, **caractérisé en ce que** les moyens d'entraînement comportent un étrier (12) solidaire d'un levier (14) monté pivotant dans le boîtier (10, 18), l'étrier (12) entourant un excentrique (20) entraîné par l'arbre (22) d'un moteur électrique (24), ledit support (32) de la surface abrasive (30) étant monté à l'extrémité du levier (14).

11. Dispositif de dermabrasion selon l'une quelconque des revendications 1 à 9, **caractérisé en ce que** le support porte-abrasif (30) est cylindrique et porte au moins une surface abrasive arquée sur sa surface cylindrique.

12. Procédé de traitement cosmétique de la peau par microépidermabrasion, utilisant le dispositif selon l'une quelconque des revendications précédentes.

13. Procédé selon la revendication 12, **caractérisé en ce qu'**on applique auparavant à la surface de la peau à traiter, un produit de nettoyage.

14. Procédé de traitement cosmétique de la peau comportant au préalable une microépidermabrasion selon la revendication 12 ou 13, suivi de l'application sur l'épiderme ainsi traité d'un produit traitant qu'on fait pénétrer dans le tissu de la peau par l'application d'un flux d'énergie électromagnétique haute fréquence et/ou par l'application de rayonnement électromagnétique laser et/ou de la lumière.

15. Procédé selon l'une quelconque des revendications 12 à 14, pour un traitement antirides, des taches, des vergetures, de l'acné, des cicatrices, d'épilation ou du cuir-chevelu.

16. Utilisation du dispositif selon l'une quelconque des revendications 1 à 11 pour la microépidermabrasion de la peau.
